(19) Europäisches Patentamt
European Patent Office
Office européen des brevets

(11) Veröffentlichungsnummer: **0 093 949**
**B1**

# (12) EUROPÄISCHE PATENTSCHRIFT

(45) Veröffentlichungstag der Patentschrift: **12.03.86**

(51) Int. Cl.⁴: **C 07 D 223/10,** C 07 D 417/12, C 07 D 401/12, A 23 K 1/16

(21) Anmeldenummer: **83104096.9**

(22) Anmeldetag: **27.04.83**

## (54) Sulfinyl- und Sulfonyl-azacycloheptan-2-one, Verfahren zu ihrer Herstellung sowie ihre Verwendung als Futterzusatzstoffe.

(30) Priorität: **08.05.82 DE 3217373**

(43) Veröffentlichungstag der Anmeldung: **16.11.83 Patentblatt 83/46**

(45) Bekanntmachung des Hinweises auf die Patenterteilung: **12.03.86 Patentblatt 86/11**

(84) Benannte Vertragsstaaten: **AT BE CH DE FR GB IT LI LU NL SE**

(56) Entgegenhaltungen: **EP - A - 0 049 505**

(73) Patentinhaber: **BAYER AG, Konzernverwaltung RP Patentabteilung, D-5090 Leverkusen 1 Bayerwerk (DE)**

(72) Erfinder: **Fengler, Gerd, Dr., Bethelstrasse 16, D-4150 Krefeld (DE)**
Erfinder: **Botta, Artur, Dr., Buschstrasse 149, D-4150 Krefeld (DE)**
Erfinder: **Scheer, Martin, Dr., Herberts-Katernberg 7, D-5600 Wuppertal 1 (DE)**
Erfinder: **Berschauer, Friedrich, Dr., Claudiusweg 9, D-5600 Wuppertal 1 (DE)**

EP 0 093 949 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Sulfinyl- und Sulfonylazacycloheptan-2-one, Verfahren zu ihrer Herstellung sowie ihre Verwendung als Mittel zur Förderung des Wachstums und der Futterverwertung bei Tieren.

Es wurde gefunden, dass die neuen Sulfinyl- und Sulfonylazacycloheptan-2-one der allgemeinen Formel (I)

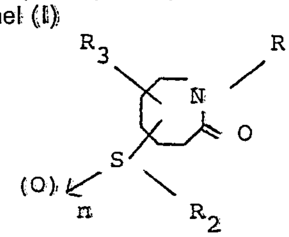

(I)

in welcher

n 1 oder 2 bedeutet,

$R_1$ für H, ggf. substituiertes $C_{1-4}$-Alkyl, ggf. substituiertes Phenyl, Acetyl, Propionyl, Benzoyl und $C_{1-4}$-Alkylcarbamoyl steht,

$R_2$ ggf. substituiertes $C_{1-18}$-Alkyl, ggf. substituiertes $C_{2-6}$-Alkenyl, ggf. substituiertes $C_{3-10}$-Cycloalkyl, ggf. substituiertes Aralkyl mit 6 oder 10 C-Atomen im Arylteil und 1-4 C-Atomen im Alkylteil, ggf. substituiertes $C_{6-10}$-Aryl oder ggf. substituiertes Heterocyclyl mit 5-7 Ringatomen und 1-3 gleichen oder verschiedenen Heteroatomen bedeutet, wobei der Sulfinyl- bzw. Sulfonylrest sich in der α- bis δ-Position befinden kann,

$R_3$ für H oder $C_{1-4}$-Alkyl steht und die α- bis ε-Position einnehmen kann;

ggf. substituierte Alkyl-, Alkenyl-, Cycloalkyl-, Aralkyl-, Aryl- und Heterocyclylreste können einen oder mehrere, gleiche oder verschiedene Reste $R_4$ tragen, wobei $R_4$ für geradkettiges oder verzweigtes Alkyl mit 1 bis 6 C-Atomen und $CF_3$, $CCl_3$ sowie für Phenyl, $CH_3O-$, $C_2H_5O-$ sowie für Phenoxy, weiterhin für $CH_3S-$, $C_2H_5S-$, für $HCO-NH-$, für Dimethylamino, Diethylamino, für $CH_3OCO$, $C_2H_5OCO$, für Halogen, $-C{\equiv}N$, COOH und für $-NH_2$ sowie $NO_2$ steht, das Wachstum und die Futterverwertung bei Tieren verbessern.

Es ist bekannt, dass es für Moleküle mit einem Asymmetriezentrum zwei Enantiomere und für Moleküle mit zwei Asymmetriezentren zwei Diastereomere und damit zwei Enantiomerenpaare gibt.

Die erfindungsgemässen Sulfinyl- bzw. Sulfonylazacycloheptan-2-one der allgemeinen Formel (I) besitzen zwei Asymmetriezentren bzw. ein Asymmetriezentrum, demgemäss sollen alle optischen Isomere und Diastereomere zum Gegenstand der vorliegenden Erfindung gehören.

Besonders bevorzugt sind Sulfinyl- und Sulfonylazacycloheptan-2-one der allgemeinen Formel (Ia)

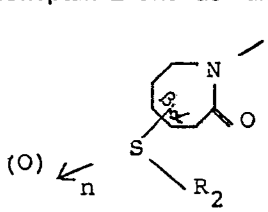

(Ia)

in welcher n und $R_2$ die oben angegebene Bedeutung haben und sich der Sulfinyl- bzw. Sulfonylrest in der α- oder β-Position befindet.

Weiterhin wurde gefunden, dass man die neuen Sulfinyl- und Sulfonylazacycloheptan-2-one der allgemeinen Formel (I) erhält, wenn man

a) Thioether der Formel (II)

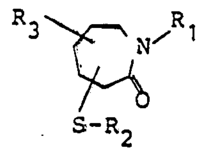

(II)

in welcher $R_1$, $R_2$ und $R_3$ die oben angegebene Bedeutung haben und der Sulfenylrest in α- bis δ-Position stehen kann, mit geeigneten Oxidationsmitteln, ggf. in Gegenwart von inerten Lösungsmitteln umsetzt.

b) Die erfindungsgemässen 3-Sulfonylazacycloheptan-2-one der Formel (III)

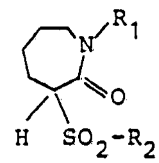

(III)

in welcher

$R_1$ und $R_2$ die oben angegebene Bedeutung besitzen, können auch durch Umsetzung von 3-Halogenazacycloheptan-2-onen der Formel (IV)

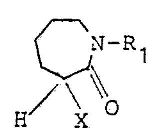

(IV)

in welcher

$R_1$ die oben angegebene Bedeutung besitzt, und

X für Halogen steht,

mit Sulfinaten der Formel (V)

$$M^{\oplus}\ {}^{\ominus}O_2S-R_2 \qquad (V)$$

in welcher

$R_2$ die oben angegebene Bedeutung hat und $M^{\oplus}$ ein Alkalimetallkation darstellt, ggf. in Gegenwart von inerten Lösungsmitteln gewonnen werden.

Die erfindungsgemässen Sulfinyl- und Sulfonylazacycloheptan-2-one zeigen eine überraschend stark ausgeprägte nutritive Wirkung, welche bei Verbindungen dieser Stoffklasse bislang noch nicht gefunden wurde, darüber hinaus sind sie weder mutagen (Ames-Test) noch östrogen wirksam und stellen somit eine Bereicherung der Technik dar.

*Verfahren a:*

Verwendet man beispielsweise 3-(Phenylthio)-azacycloheptan-2-on und m-Chlorperbenzoesäure als Ausgangsstoffe, so kann der Reaktionsablauf durch das folgende Formelschema wiedergegeben werden

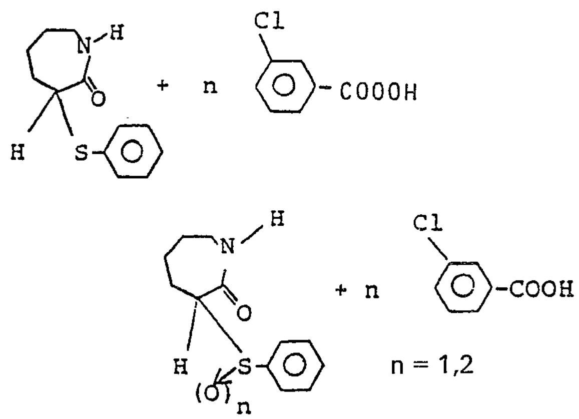

n = 1,2

In der allgemeinen Formel (I) stehen als ggf. substituiertes $C_{1-4}$-Alkyl $R_1$ und $R_3$ geradkettige oder verzweigte Alkylreste mit bis zu 4 Kohlenstoffatomen. Beispielhaft seien ggf. substituiertes Methyl, Ethyl, n- und i-Propyl, n-, i- und t-Butyl genannt.

Alkylcarbomyl $R_1$ steht für Carbamoylreste mit bis zu 4, insbesondere bis zu 2 Kohlenstoffatomen im Alkylteil, beispielsweise seien Methyl- und Ethylcarbamoyl genannt.

Ggf. substituiertes Alkyl $R_2$ steht für geradkettiges oder verzweigtes Alkyl mit 1-18, vorzugsweise 1-12 Kohlenstoffatomen. Beispielhaft seien ggf. substituiertes Methyl, Ethyl, n- und i-Propyl, n-, i- und t-Butyl, Pentyl, Hexyl, Dodecyl genannt.

Als ggf. substituierte Alkenylreste $R_2$ kommen geradkettige oder verzweigte Alkenylreste mit bis zu 6, bevorzugt bis zu 4 Kohlenstoffatomen in Frage. Beispielhaft seien genannt: Ethenyl, Propenyl-(1), Propenyl-(2), Butenyl-(3).

Ggf. substituiertes Cycloalkyl $R_2$ ist mono-, bi- oder tricyclisch und enthält vorzugsweise 3 bis 10, insbesondere 3 bis 7 C-Atome. Beispielhaft seien ggf. substituiertes Cyclopropyl, Cyclobutyl, Cyclopentyl, Cyclohexyl, Cyclohexenyl, Cycloheptyl, Bicyclo-[2,2,1]-heptyl und Adamantyl genannt.

Als ggf. substituiertes Aralkyl $R_2$ steht ggf. im Arylteil und/oder Alkylteil substituiertes Aralkyl mit vorzugsweise 6 oder 10, insbesondere 6 Kohlenstoffatomen im Arylteil und vorzugsweise 1 bis 4, insbesondere 1 oder 2 Kohlenstoffatomen im Alkylteil, wobei der Alkylteil geradkettig oder verzweigt sein kann. Beispielhaft seien ggf. substituiertes Benzyl und Phenylethyl genannt.

Als ggf. substituiertes Aryl $R_2$ steht Aryl mit vorzugsweise 6 bis 10 Kohlenstoffatomen im Arylteil. Beispielhaft seien ggf. substituiertes Phenyl oder Naphthyl genannt. Substituenten im Phenylring stehen in o-, m- oder p-Stellung.

Als ggf. substituiertes Heterocyclyl $R_2$ stehen heteroparaffinische, heteroaromatische oder heteroolefinische 5- bis 7gliedrige, vorzugsweise 5- oder 6gliedrige Ringe mit vorzugsweise 1 bis 3, insbesondere 1 oder 2 gleichen oder verschiedenen Heteroatomen. Als Heteroatome stehen Sauerstoff, Schwefel oder Stickstoff. Als Beispiele seien ggf. substituiertes Thienyl, Furyl, Oxazolyl, Isoxazolyl, Thiazolyl, Isothiazolyl, Pyrrolyl, Imidazolyl, Pyrazolyl, Oxdiazolyl, Thiatriazolyl, Tetrazolyl, Pyridyl, Pyrazinyl, Pyrimidinyl, Tetrahydrofuranyl, Dioxanyl, Pyrrolidinyl, Piperidinyl und Morpholinyl, genannt.

Es ist auch möglich, dass die heterocyclischen Ringe an einen oder mehrere Reste aus der Benzolreihe kondensiert sind.

Ggf. substituierte Alkyl-, Alkenyl-, Cycloalkyl-, Aralkyl-, Aryl- und Heterocyclylreste können einen oder mehrere, vorzugsweise 1 bis 3, insbesondere 1 oder 2 gleiche oder verschiedene Reste $R_4$ tragen, wobei $R_4$ für geradkettiges oder verzweigtes Alkyl mit vorzugsweise 1 bis 6, insbesondere 1 bis 4 C-Atomen, z. B. Methyl, Ethyl, n- und i-Propyl, n-, i- und t-Butyl und $CF_3$, $CCl_3$ sowie für Aryl, z. B. Phenyl, $CH_3O-$, $C_2H_5O-$ sowie für Aryloxy, z. B. Phenoxy, weiterhin für $CH_3S-$, $C_2H_5S-$, für $HCO-NH-$, für Dimethylamino, Diethylamino, für $CH_3O-CO-$ und $C_2H_5O-CO-$, für Halogen, bevorzugt Fluor, Chlor, Brom, $-C{\equiv}N$, COOH und für $-NH_2$ sowie $NO_2$ steht.

Die erfindungsgemäss verwendbaren Thioether der Formel (II) sind teilweise bekannt oder lassen sich nach bekannten Verfahren herstellen („Faserforsch. u. Textiltechn.", *14*, 368-374 [1963], „Aust. J. Chem. ", *34*, 569-81 [1981]).

Die erfindungsgemäss verwendbaren Oxidationsmittel sind ebenfalls bekannt, beispielhaft seien genannt Wasserstoffperoxid, Perameisensäure, Peressigsäure, Perbenzosäure, m-Chlorperbenzoesäure, Natriummetaperjodat sowie Luftsauerstoff. Weitere geeignete Oxidationsmittel sind in C. Ferri, „Reaktionen der organischen Synthese" (G. Thieme Verlag, Stuttgart 1978, S. 470) zu finden.

Als Verdünnungsmittel kommen alle inerten organischen Lösungsmittel in Frage. Hierzu gehören vorzugsweise chlorierte Kohlenwasserstoffe, wie Methylenchlorid, Chloroform, Tetrachlorkohlenstoff, 1,2-Dichlorethan, 1,2,2-Trichlorethan und Chlorbenzol, Alkohole, vorzugsweise Methanol, Ethanol sowie Isopropanol, niedere Fettsäuren, bevorzugt Ameisensäure, Essigsäure und Propionsäure und Wasser. Das erfindungsgemässe Verfahren kann durchgeführt werden in Gegenwart von ausschliesslich einem oder mehreren organischen Lösungsmitteln oder Wasser und einem oder mehreren mit Wasser nicht mischbaren Lösungsmitteln.

Die Reaktionstemperaturen können in einem grösseren Bereich variiert werden. Im allgemeinen arbeitet man zwischen etwa −20 und etwa +100° C, vorzugsweise zwischen 0 und +60° C. Die Umsetzung kann bei Normaldruck, aber auch bei vermindertem und erhöhtem Druck ausgeführt werden. Im allgemeinen arbeitet man bei Normaldruck.

Bei der Durchführung des erfindungsgemässen Verfahrens setzt man zur Darstellung der Sulfoxide ((I), n=1) bzw. der Sulfone ((I), n=2) die jeweils angemessene Menge an Oxidationsmittel ein.

Die Aufarbeitung der Reaktionsansätze zur Isolierung der erfindungsgemässen Verbindungen erfolgt durchweg in allgemein bekannter Art und Weise.

*Verfahren b:*

Verwendet man beispielsweise α-Bromcaprolactam und Natrium-(4-chlorphenyl)sulfinat als Ausgangsstoffe, so kann der Reaktionsablauf durch das folgende Formelschema wiedergegeben werden:

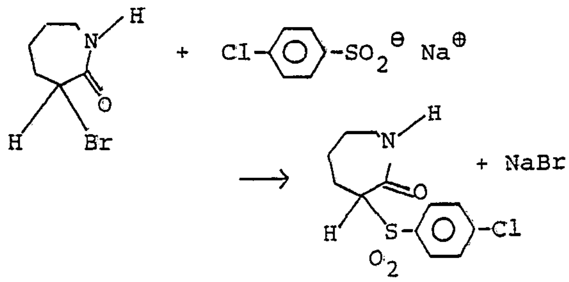

In der allgemeinen Formel (IV) steht X für Halogen, bevorzugt Chlor, Brom sowie Iod, insbesondere für Chlor und Brom.

$R_1$ hat die oben angegebene Bedeutung.

Die erfindungsgemäss verwendbaren α-Halogencaprolactame sind bekannt oder können nach bekannten Verfahren hergestellt werden (z. B. „J. Amer. Chem. Soc.", *80*, 6238-6244 [1958]).

In der allgemeinen Formel (V) hat $R_2$ die oben angegebene Bedeutung, $M^{\oplus}$ steht für ein Alkalikation, vorzugsweise für das Natrium- bzw. Kaliumkation.

Die erfindungsgemäss verwendbaren Alkalisulfinate sind bekannt (Houben-Weyl, G. Thieme Verlag, Stuttgart, 1955, Bd. IX, S. 289-342).

Als Verdünnungsmittel kommen alle inerten organischen Lösungsmittel in Frage. Hierzu gehören vorzugsweise Alkohol wie Methanol, Ethanol und Isopropanol, Ether wie Dioxan oder Tetrahydrofuran, dipolar aprotische Solventien wie Dimethylsulfoxid, Dimethylformamid sowie N-Methylpyrrolidon und Wasser.

Das erfindungsgemässe Verfahren kann durchgeführt werden in Gegenwart von ausschliesslich einem oder mehreren Lösungsmitteln oder Wasser und einem oder mehreren mit Wasser nicht mischbaren Lösungsmitteln.

Die Reaktionstemperaturen können in einem grösseren Bereich variiert werden. Im allgemeinen arbeitet man zwischen etwa 0 und +250, vorzugsweise zwischen +50 und +190° C. Die Umsetzung kann bei Normaldruck, aber auch bei vermindertem und erhöhtem Druck ausgeführt werden. Im allgemeinen arbeitet man bei Normaldruck.

Bei der Durchführung des erfindungsgemässen Verfahrens setzt man die Ausgangsstoffe in äquimolaren Mengen ein, es kann jedoch zweckmässig sein, das Alkalisulfinat der allgemeinen Formel (V) im Überschuss zu verwenden, um die Reaktionsgeschwindigkeit zu erhöhen.

Die Aufarbeitung der Reaktionsansätze zur Isolierung der erfindungsgemässen Verbindungen erfolgt in durchweg allgemein bekannter Art und Weise.

Als neue Wirkstoffe seien im einzelnen beispielhaft genannt:

3-(Butylsulfinyl)azacycloheptan-2-on
4-(Butylsulfinyl)azacycloheptan-2-on
5-(Butylsulfinyl)azacycloheptan-2-on
6-(Butylsulfinyl)azacycloheptan-2-on
3-[(2-Hydroxyethyl)sulfinyl]azacycloheptan-2-on
3-[(Carboxymethyl)sulfinyl]azacycloheptan-2-on
4-[(Carboxymethyl)sulfinyl]azacycloheptan-2-on
5-[(Carboxymethyl)sulfinyl]azacycloheptan-2-on
6-[(Carboxymethyl)sulfinyl]azacycloheptan-2-on
3-[(2-Carboxy-2-aminoethyl)sulfinyl]azacycloheptan-2-on
4-[(2-Carboxy-2-aminoethyl)sulfinyl]azacycloheptan-2-on
5-[(2-Carboxy-2-aminoethyl)sulfinyl]azacycloheptan-2-on
6-[(2-Carboxy-2-aminoethyl)sulfinyl]azacycloheptan-2-on
3-(Dodecylsulfinyl)azacycloheptan-2-on
3-(Cyclohexylsulfinyl)azacycloheptan-2-on
4-(Cyclopentylsulfinyl)azacycloheptan-2-on
3-(Benzylsulfinyl)azacycloheptan-2-on
4-(Benzylsulfinyl)azacycloheptan-2-on
5-(Benzylsulfinyl)azacycloheptan-2-on
6-(Benzylsulfinyl)azacycloheptan-2-on
3-(Allylsulfinyl)azacycloheptan-2-on
4-(Allylsulfinyl)azacycloheptan-2-on
5-(Allylsulfinyl)azacycloheptan-2-on
6-(Allylsulfinyl)azacycloheptan-2-on
3-[(3-Chlor-2-propenyl)sulfinyl]azacycloheptan-2-on
4-[(3-Chlor-2-propenyl)sulfinyl]azacycloheptan-2-on
3-[(3,3-Dichlor-2-propenyl)sulfinyl]azacycloheptan-2-on
3-(Phenylsulfinyl)azacycloheptan-2-on
4-(Phenylsulfinyl)azacycloheptan-2-on
3-[(4-Chlorphenyl)sulfinyl]azacycloheptan-2-on
4-[(4-Chlorphenyl)sulfinyl]azacycloheptan-2-on
5-[(4-Chlorphenyl)sulfinyl]azacycloheptan-2-on
6-[(4-Chlorphenyl)sulfinyl]azacycloheptan-2-on
3-[(3-Chlorphenyl)sulfinyl]azacycloheptan-2-on
4-[(3-Chlorphenyl)sulfinyl]azacycloheptan-2-on
5-[(3-Chlorphenyl)sulfinyl]azacycloheptan-2-on
6-[(3-Chlorphenyl)sulfinyl]azacycloheptan-2-on
3-[(2-Chlorphenyl)sulfinyl]azacycloheptan-2-on
4-[(2-Chlorphenyl)sulfinyl]azacycloheptan-2-on
5-[(2-Chlorphenyl)sulfinyl]azacycloheptan-2-on
6-[(2-Chlorphenyl)sulfinyl]azacycloheptan-2-on
3-[(3,4-Dichlorphenyl)sulfinyl]azacycloheptan-2-on

4-[(3,4-Dichlorphenyl)sulfinyl]azacycloheptan-2-on
3-[(2,4-Dichlorphenyl)sulfinyl]azacycloheptan-2-on
4-[(2,4-Dichlorphenyl)sulfinyl]azacycloheptan-2-on
3-[(3,5-Dichlorphenyl)sulfinyl]azacycloheptan-2-on
4-[(3,5-Dichlorphenyl)sulfinyl]azacycloheptan-2-on
3-[(3-Fluor-4-chlorphenyl)sulfinyl]azacycloheptan-2-on
4-[(3-Fluor-4-chlorphenyl)sulfinyl]azacycloheptan-2-on
3-[(3-Trifluormethylphenyl)sulfinyl]azacycloheptan-2-on
4-[(3-Trifluormethylphenyl)sulfinyl]azacycloheptan-2-on
3-[(4-Methylphenyl)sulfinyl]azacycloheptan-2-on
4-[(4-Methylphenyl)sulfinyl]azacycloheptan-2-on
3-[(4-Nitrophenyl)sulfinyl]azacycloheptan-2-on
4-[(4-Nitrophenyl)sulfinyl]azacycloheptan-2-on
3-[(4-Bromphenyl)sulfinyl]azacycloheptan-2-on
4-[(4-Bromphenyl)sulfinyl]azacycloheptan-2-on
3-[(4-Aminophenyl)sulfinyl]azacycloheptan-2-on
4-[(4-Aminophenyl)sulfinyl]azacycloheptan-2-on
3-[(2-Carboxyphenyl)sulfinyl]azacycloheptan-2-on
4-[(2-Carboxyphenyl)sulfinyl]azacycloheptan-2-on
3-[(4-Cyanophenyl)sulfinyl]azacycloheptan-2-on
4-[(4-Cyanophenyl)sulfinyl]azacycloheptan-2-on
3-[(3-Ethoxy-4-chlorphenyl)sulfinyl]azacycloheptan-2-on
4-[(3-Ethoxy-4-chlorphenyl)sulfinyl]azacycloheptan-2-on
3-[(4-Dimethylaminophenyl)sulfinyl]azacycloheptan-2-on
1-Methyl-3-allylsulfinylazacycloheptan-2-on
1-Acetyl-3-allylsulfinylazacycloheptan-2-on
1-Methylcarbamoyl-3-allylsulfinylazacycloheptan-2-on
1-Ethyl-3-[(4-chlorphenyl)sulfinyl]azacycloheptan-2-on
1-Phenyl-3-[(4-chlorphenyl)sulfinyl]azacycloheptan-2-on
1-Phenyl-4-[(4-chlorphenyl)sulfinyl]azacycloheptan-2-on
1-Acetyl-3-[(4-chlorphenyl)sulfinyl]azacycloheptan-2-on
1-Acetyl-4-[(4-chlorphenyl)sulfinyl]azacycloheptan-2-on
1-Propionyl-3-[(4-chlorphenyl)sulfinyl]azacycloheptan-2-on
1-Propionyl-4-[(4-chlorphenyl)sulfinyl]azacycloheptan-2-on
1-Methylcarbamoyl-3-[(4-chlorphenyl)sulfinyl]azacycloheptan-2-on
1-Methylcarbamoyl-4-[(4-chlorphenyl)sulfinyl]azacycloheptan-2-on
5-(tert.-Butyl)-3-[(4-chlorphenyl)sulfinyl]azacycloheptan-2-on
3-(Butylsulfonyl)azacycloheptan-2-on
5-(Butylsulfonyl)azacycloheptan-2-on
3-(Allylsulfonyl)azacycloheptan-2-on
3-(Benzylsulfonyl)azacycloheptan-2-on
3-(Phenylsulfonyl)azacycloheptan-2-on
4-(Phenylsulfonyl)azacycloheptan-2-on
6-(Phenylsulfonyl)azacycloheptan-2-on
3-[(4-Chlorphenyl)sulfonyl]azacycloheptan-2-on
4-[(4-Chlorphenyl)sulfonyl]azacycloheptan-2-on
5-[(4-Chlorphenyl)sulfonyl]azacycloheptan-2-on
6-[(4-Chlorphenyl)sulfonyl]azacycloheptan-2-on
3-[(3-Chlorphenyl)sulfonyl]azacycloheptan-2-on
4-[(3-Chlorphenyl)sulfonyl]azacycloheptan-2-on
5-[(3-Chlorphenyl)sulfonyl]azacycloheptan-2-on
6-[(3-Chlorphenyl)sulfonyl]azacycloheptan-2-on
3-[(2-Chlorphenyl)sulfonyl]azacycloheptan-2-on
4-[(2-Chlorphenyl)sulfonyl]azacycloheptan-2-on
5-[(2-Chlorphenyl)sulfonyl]azacycloheptan-2-on
6-[(2-Chlorphenyl)sulfonyl]azacycloheptan-2-on
3-[(2,4-Dichlorphenyl)sulfonyl]azacycloheptan-2-on
4-[(2,4-Dichlorphenyl)sulfonyl]azacycloheptan-2-on
3-[(3,4-Dichlorphenyl)sulfonyl]azacycloheptan-2-on
4-[(3,4-Dichlorphenyl)sulfonyl]azacycloheptan-2-on
3-[(3,5-Dichlorphenyl)sulfonyl]azacycloheptan-2-on
4-[(3,5-Dichlorphenyl)sulfonyl]azacycloheptan-2-on
3-[(3-Trifluormethylphenyl)sulfonyl]azacycloheptan-2-on
4-[(3-Trifluormethylphenyl)sulfonyl]azacycloheptan-2-on
3-[(4-Methylphenyl)sulfonyl]azacycloheptan-2-on
4-[(4-Methylphenyl)sulfonyl]azacycloheptan-2-on
3-[(4-Nitrophenyl)sulfonyl]azacycloheptan-2-on
4-[(4-Nitrophenyl)sulfonyl]azacycloheptan-2-on
3-[(4-Aminophenyl)sulfonyl]azacycloheptan-2-on
4-[(4-Aminophenyl)sulfonyl]azacycloheptan-2-on
3-[(2-Carboxyphenyl)sulfonyl]azacycloheptan-2-on
4-[(2-Carboxyphenyl)sulfonyl]azacycloheptan-2-on
3-[(4-Cyanophenyl)sulfonyl]azacycloheptan-2-on
4-[(4-Cyanophenyl)sulfonyl]azacycloheptan-2-on
3-[(4-Fluorphenyl)sulfonyl]azacycloheptan-2-on
4-[(4-Fluorphenyl)sulfonyl]azacycloheptan-2-on
1-Acetyl-3-[(4-chlorphenyl)sulfonyl]azacycloheptan-2-on
1-Acetyl-4-[(4-chlorphenyl)sulfonyl]azacycloheptan-2-on
1-Propionyl-3-[(4-chlorphenyl)sulfonyl]azacycloheptan-2-on
1-Propionyl-4-[(4-chlorphenyl)sulfonyl]azacycloheptan-2-on
1-Methylcarbamoyl-3-[(4-chlorphenyl)sulfonyl]azacycloheptan-2-on
1-Methylcarbamoyl-4-[(4-chlorphenyl)sulfonyl]azacycloheptan-2-on
1-Ethyl-3-[(3,4-dichlorphenyl)sulfonyl]azacycloheptan-2-on
1-Ethyl-4-[(3,4-dichlorphenyl)sulfonyl]azacycloheptan-2-on
1-Acetyl-3-[(3,4-dichlorphenyl)sulfonyl]azacycloheptan-2-on
1-Acetyl-4-[(3,4-dichlorphenyl)sulfonyl]azacycloheptan-2-on
1-Propionyl-3-[(3,4-dichlorphenyl)sulfonyl]-azacycloheptan-2-on
1-Propionyl-4-[(3,4-dichlorphenyl)sulfonyl]-azacycloheptan-2-on
3-[(2-Benzothiazolyl)sulfinyl]azacycloheptan-2-on
4-[(2-Benzothiazolyl)sulfinyl]azacycloheptan-2-on
3-[(2-Benzothiazolyl)sulfonyl]azacycloheptan-2-on
4-[(2-Benzothiazolyl)sulfonyl]azacycloheptan-2-on
3-[(2-Pyridyl)sulfinyl]azacycloheptan-2-on
4-[(2-Pyridyl)sulfinyl]azacycloheptan-2-on
3-[(2-Pyridyl)sulfonyl]azacycloheptan-2-on
4-[(2-Pyridyl)sulfonyl]azacycloheptan-2-on
3-[(2-(3-Chlortetrahydrofuranyl))sulfinyl]azacycloheptan-2-on
3-[(2-(3-Chlortetrahydrofuranyl))sulfonyl]azacycloheptan-2-on
3-[(2-(3-Chlorchinoxalinyl))sulfinyl]azacycloheptan-2-on
3-[(2-(3-Chlorchinoxalinyl))sulfonyl]azacycloheptan-2-on
3-[(3-Triazolyl-1,2,4)sulfinyl]azacycloheptan-2-on
3-[(3-Triazolyl-1,2,4)sulfonyl]azacycloheptan-2-on

Die erfindungsgemässen Azacycloheptan-2-one der Formel (I) weisen überraschenderweise die Eigenschaft auf, bei Tieren das Wachstum zu fördern und zu beschleunigen, so dass diese Verbindungen in allen Bereichen der Tierzucht und Tierhaltung zu den genannten Zwecken eingesetzt werden können.

Die Wirksamkeit der erfindungsgemäss verwendeten Verbindungen ist hierbei weitgehend unabhängig von der Art und dem Geschlecht der Tiere. Besonders wertvoll erweisen sich die erfindungsgemässen Azacycloheptan-2-one der Formel (I) bei der Aufzucht und Haltung von Jung- und Masttieren. Als Tiere, bei denen die Verbindung zur Förderung und Beschleunigung des Wachstums eingesetzt werden kann, seien beispielsweise folgende Nutz- und Ziertiere genannt:

Warmblüter wie Rinder, Schweine, Pferde, Schafe, Ziegen, Katzen, Hunde, Kaninchen, Pelztiere, z. B. Nerze und Chinchilla, Geflügel, z. B. Hühner, Gänse, Enten, Truthähne, Broiler, Tauben, Papageien und Kanarienvögel und Kaltblüter, wie Fische, z. B. Karpfen und Reptilien, z. B. Schlangen.

Bevorzugte Anwendung finden Azacycloheptan-2-one der Formel (I) bei der Aufzucht und Haltung von Wiederkäuern wie Kälber, Ziegen, Schafe sowie bei Schweinen und Küken.

Die Menge von Azacycloheptan-2-onen der Formel (I), die den Tieren zur Erreichung des gewünschten Effektes verabreicht wird, kann weitgehend variiert werden. Sie liegt vorzugsweise bei etwa 0,5 bis 500, insbesondere 1 bis 100 mg/kg Körpergewicht/d. Die Dauer der Verabreichung kann von wenigen Stunden oder Tagen bis zu mehreren Jahren betragen. Die passende Menge Wirkstoff sowie die passende Dauer der Verabreichung hängen insbesondere von der Art, dem Alter, dem Geschlecht, dem Gesundheitszustand und der Art der Haltung der Tiere ab und sind durch jeden Fachmann leicht zu ermitteln.

Die Verbindungen werden den Tieren nach den üblichen Methoden verabreicht. Die Art der Verabreichung hängt insbesondere von der Art, dem Verhalten und dem Gesundheitszustand der Tiere ab. So kann die Verabreichung einmal oder mehrmals täglich in regelmässigen oder unregelmässigen Abständen oral oder parenteral erfolgen. Aus Zweckmässigkeitsgründen ist in den meisten Fällen eine orale Verabreichung, insbesondere im Rhythmus der Nahrungs- und/oder Getränkeaufnahme der Tiere, vorzuziehen.

Die Verbindungen können als reine Stoffe oder in formulierter Form, also in Mischung mit nichttoxischen inerten Trägerstoffen, als solche sind feste, halbfeste oder flüssige Verdünnungsmittel, Füllstoffe und Formulierungshilfsmittel jeder Art zu verstehen, verabreicht werden.

Azacycloheptan-2-one der Formel (1) können ggf. in formulierter Form auch zusammen mit pharmazeutischen Wirkstoffen, Mineralsalzen, Spurenelementen, Vitaminen, Eiweissstoffen, Fetten, Farbstoffen und/oder Geschmacksstoffen in geeigneter Form verabreicht werden.

Empfehlenswert ist die orale Verabreichung zusammen mit dem Futter und/oder Trinkwasser, wobei je nach Bedarf die Wirkstoffe der Gesamtmenge oder nur Teilen des Futters und/oder Trinkwasser zugegeben werden.

Die Verbindungen können nach üblichen Methoden durch einfaches Mischen als reine Stoffe, vorzugsweise in fein verteilter Form oder in formulierter Form in Mischung mit essbaren nichttoxischen Trägerstoffen, ggf. auch in Form eines Prämix oder eines Futterkonzentrates, dem Futter und/oder Trinkwasser beigefügt werden.

Das Futter und/oder Trinkwasser kann beispielsweise den erfindungsgemässen Wirkstoff in einer Konzentration von etwa 5 bis 500, insbesondere 5 bis 100 ppm enthalten. Die optimale Höhe der Konzentration des Wirkstoffes in dem Futter und/oder Trinkwasser ist insbesondere abhängig von der Menge der Futter- und/oder Trinkwasseraufnahme der Tiere und kann durch jeden Fachmann leicht ermittelt werden.

Die Art des Futters und seine Zusammensetzung ist hierbei ohne Belang. Es können alle gebräuchlichen, handelsüblichen oder speziellen Futterzusammensetzungen verwendet werden, die vorzugsweise das übliche, für eine ausgewogene Ernährung notwendige Gleichgewicht aus Energie- und Aufbaustoffen einschliesslich Vitaminen und Mineralstoffen enthalten. Das Futter kann sich beispielsweise zusammensetzen aus pflanzlichen Stoffen, z. B. Heu, Rüben, Getreide, Getreidenebenprodukten, tierischen Stoffen, z. B. Fleisch, Fetten, Knochenmehl, Fischprodukten, Vitaminen, z. B. Vitamin A, D-Komplex und B-Komplex, Proteinen, Aminosäuren, z. B. DL-Methionin und anorganischen Stoffen, z. B. Kalk und Kochsalz.

Futterkonzentrate enthalten Azacycloheptan-2-one der Formel (I) neben essbaren Stoffen, z. B. Roggenmehl, Maismehl, Sojabohnenmehl oder Kalk, ggf. mit weiteren Nähr-und Aufbaustoffen sowie Proteinen, Mineralsalzen und Vitaminen. Sie können nach den üblichen Mischmethoden hergestellt werden.

Vorzugsweise in Prämixen und Futterkonzentrationen können die Wirkstoffe ggf. auch durch seine Oberfläche bedeckende geeignete Mittel, z. B. mit nichttoxischen Wachsen oder Gelatine vor Luft, Licht und/oder Feuchtigkeit geschützt werden.

Beispiel für die Zusammensetzung eines Kükenaufzuchtfutters, das Azacycloheptan-2-one der Formel (I) enthält.

200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung und 2,5 g Wirkstoffprämix ergeben nach sorgfältigem Mischen 1 kg Futter.

Die Vitamin-Mineral-Mischung besteht aus:

6000 I.E. Vitamin A, 1000 I.E. Vitamin $D_3$, 10 mg Vitamin E, 1 mg Vitamin $K_3$, 3 mg Riboflavin, 2 mg Pyridoxin, 20 mg Vitamin $B_{12}$, 5 mg Calciumpantothenat, 30 mg Nikotinsäure, 200 mg Cholinchlorid, 200 mg $MnSO_4 \times H_2O$, 140 mg $ZnSO_4 \times 7 H_2O$, 100 mg $FeSO_4 \times 7 H_2O$ und 20 mg $CuSO_4 \times 5 H_2O$.

Der Wirkstoffprämix enthält Azacycloheptan-2-one der Formel (I) in der gewünschten Menge, z. B. 100 mg und zusätzlich 1 g DL-Methionin sowie so viel Sojabohnenmehl, dass 2,5 g Prämix entstehen.

Beispiel für die Zusammensetzung eines Schweineaufzuchtfutters, dass den erfindungsgemässen Wirkstoff enthält:

630 g Futtergetreideschrot (zusammengesetzt aus 200 g Mais, 150 g Gerste-, 150 g Hafer- und 130 g Weizenschrot), 80 g Fischmehl, 60 g Sojaschrot, 60 g Tapiokamehl, 38 g Eierhefe, 50 g Vitamin-Mineral-Mischung für Schweine (Zusammensetzung z. B. wie beim Kükenfutter), 30 g Leinkuchenmehl, 30 g Maiskleberfutter, 10 g Sojaöl, 10 g Zuckerrohrmelasse und 2 g Wirkstoffprämix (Zusammensetzung z. B. wie beim Kükenfutter) ergeben nach sorgfältigem Mischen 1 kg Futter.

Die angegebenen Futtergemische sind vorzugsweise zur Aufzucht und Mast von Küken bzw. Schweinen abgestimmt, sie können jedoch in gleicher oder ähnlicher Zusammensetzung auch zur Aufzucht und Mast anderer Tiere verwendet werden.

14tätige Fütterungsversuche an Küken und 6wöchige Fütterungsversuche an Broilern, die 5 bis 500 ppm Verbindung der Formel (I) mit dem Futter enthielten, zeigten deutliche Gewichtszunahme bei den mit Azacycloheptan-2-onen der Formel (I) behandelten Tiere im Vergleich zu den ohne Zusatz von Azacycloheptan-2-onen der Formel (I) ernährten Tieren.

*Beispiel 1:*

*Herstellung von 3-[(4-Chlorphenyl)thio]azacycloheptan-2-on*

In 500 ml Methanol legt man 54 g (1,0 Mol) Natriummethylat und 144,6 g (1,0 Mol) 4-Chlorthiophenol vor und tropft bei Raumtemperatur eine methanolische Lösung von 192 g (1,0 Mol) α-Bromcaprolactam zu. Nach Abklingen der exothermen Reaktion rührt man noch 3 Std. bei Raumtemperatur, saugt dann das ausgefallene Natriumbromid ab und engt die Mutterlauge im Vakuum ein. Die ausgefallene Kristalle werden abgesaugt.

Ausbeute: 250 g (= 98% d.Th.)

Fp.: 176° C

*Beispiel 2:*

*Herstellung von 5-(Buthylthio)azacycloheptan-2-on*

In 100 ml Ethanol legt man 6,8 g (0,1 Mol) Natriumethylat und 9 g (0,1 Mol) Butanthiol vor und gibt bei Raumtemperatur 14,8 g (0,1 Mol) 5-Chlorazacycloheptan-2-on zu. Nach 10 Std. Rückflusskochen wird das ausgefallene Natriumchlorid abgesaugt, die Mutterlauge im Vakuum eingeengt und der Rückstand destilliert.

Ausbeute: 10,5 g (= 52% d.Th.)

Kp.: 120° C/0,9 mbar

*Beispiel 3:*

*Herstellung von 1-(Methylcarbamoyl)-3-[(4-chlorphenyl)thio]azacycloheptan-2-on*

25,6 g (0,1 Mol) 3-[(4-Chlorphenyl)thio]azacycloheptan-2-on werden zusammen mit 5,7 g

(0,1 Mol) Methylisocyanat und 100 mg Dibutylzinndilaurat in 150 ml absolutem Toluol 5 Std. am Rückfluss gekocht. Nach dem Abkühlen wird das Solvens im Vakuum abgezogen, der ölige Rückstand wird für weitere Umsetzungen verwendet.

*Beispiel 4:*

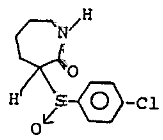

Man gibt zu einer Lösung von 25,6 g (0,1 Mol) 3-[(4-Chlorphenyl)thio]azacycloheptan-2-on in 200 ml Eisessig 11 g 30%iges Wasserstoffperoxid und lässt das Reaktionsgemisch 48 Std. bei Raumtemperatur stehen.

Anschliessend wird der Eisessig im Vakuum abgezogen und der Rückstand aus Methanol umkristallisiert.

Ausbeute: 21 g (= 72% d.Th.)
Fp.: 185-189° C (Methanol)

Analog wurden dargestellt:

| Beispiel Nr. | Formel | Ausbeute (%) | Kp. (° C/mbar) oder Fp. (° C) |
|---|---|---|---|
| 5 | 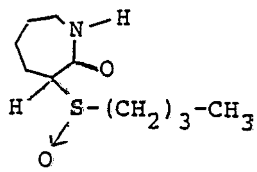 | 78 | 119 |
| 6 | 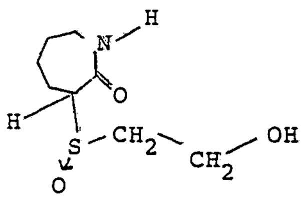 | 73 | 166-168 |
| 7 | 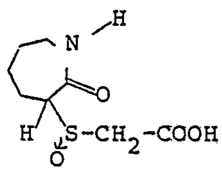 | 68 | 144-145 |
| 8 | 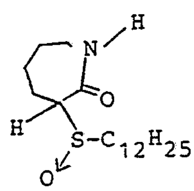 | 56 | 130-131 |
| 9 | 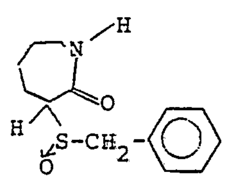 | 86 | 158-162 |
| 10 | 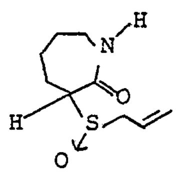 | 67 | 98-100 |
| 11 | 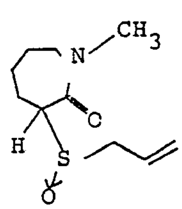 | 58 | 115 (0,7) |

| Beispiel Nr. | Formel | Ausbeute (%) | Kp. (° C/mbar) oder Fp. (° C) |
|---|---|---|---|
| 12 | 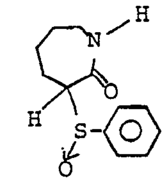 | 91 | 156-158 |
| 13 | 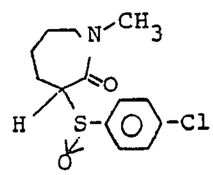 | 85 | 162-164 |
| 14 | 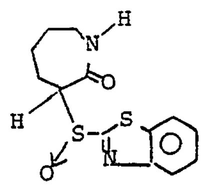 | 93 | 187-189 |
| 15 | 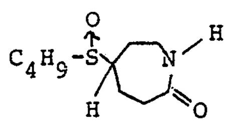 | 48 | 135 (0,9) |
| 16 | 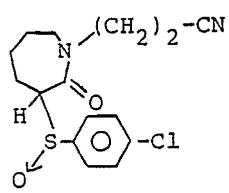 | 79 | 143-148 |
| 17 | 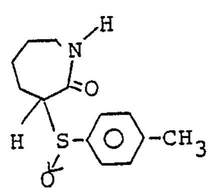 | 83 | 176-177 |
| 18 | 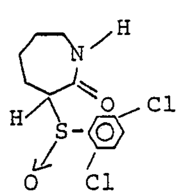 | 62 | 178-180 |

| Beispiel Nr. | Formel | Ausbeute (%) | Kp. (° C/mbar) oder Fp. (° C) |
|---|---|---|---|
| 19 | 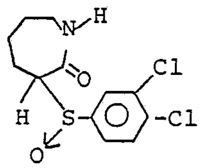 | 91 | 180-182 |
| 20 | 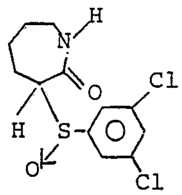 | 87 | 155-156 |
| 21 | 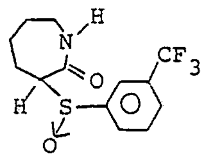 | 81 | 153-155 |
| 22 | 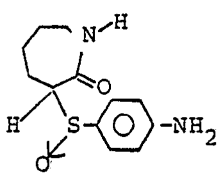 | 54 | 175-179 |
| 23 | 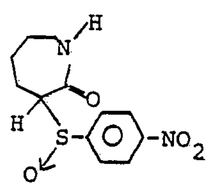 | 58 | 173-176 |
| 24 | 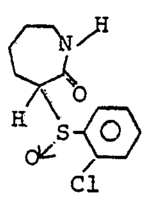 | 63 | 179-180 |
| 25 | 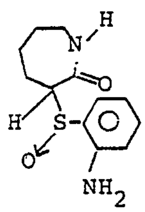 | 38 | 101-105 (Zers.) |

| Beispiel Nr. | Formel | Ausbeute (%) | Kp. (° C/mbar) oder Fp. (° C) |
|---|---|---|---|
| 26 | 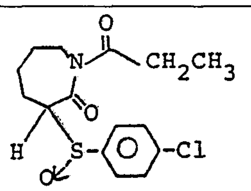 | 47 | 158-159 |
| 27 | 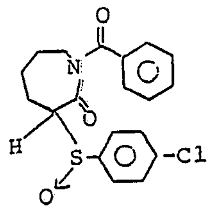 | 62 | 126-129 |

*Beispiel 28:*

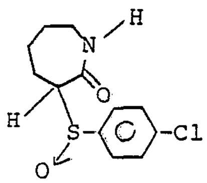

Zu 25,6 g (0,1 Mol) 3-[(4-Chlorphenyl)thio]azacycloheptan-2-on in 200 ml Chloroform werden bei Raumtemperatur 20 g m-Chlorperbenzoesäure (80-90%ig) portionsweise zugesetzt. Nach Abklingen der exothermen Reaktion wird noch 14 Std. bei Raumtemperatur gerührt und dann mit Natriumhydrogencarbonatlösung neutralisiert. Die organische Phase wird abgetrennt und eingeengt. Der kristalline Rückstand wird aus Methanol umkristallisiert.

Ausbeute: 24,5 g (= 90% d.Th.)
Fp.: 189-192° C

Analog wurden dargestellt:

| Beispiel Nr. | Formel | Ausbeute (%) | Kp. (° C/mbar) oder Fp. (° C) |
|---|---|---|---|
| 29 | 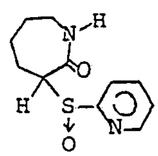 | 53 | 147-148 |
| 30 | 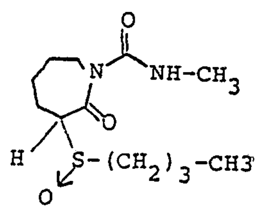 | 59 | 120 |
| 31 | 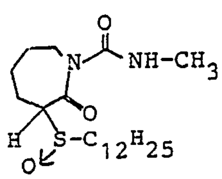 | 47 | 59 |
| 32 | 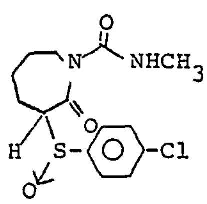 | 52 | 132-136 |

*Beispiel 33:*

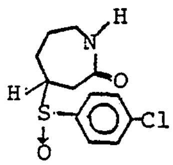

Zur Lösung von 25,6 g (0,1 Mol) 4-[(4-Chlorphenyl)thio]azacycloheptan-2-on in 100 ml Methanol gibt man 21,4 g (0,1 Mol) Natriummetaperiodat und rührt 100 Std. bei Raumtemperatur. Anschliessend wird der Niederschlag abgesaugt, die Mutterlauge eingeengt und der Rückstand kristallisiert.

Ausbeute: 18 g (= 66% d.Th)
Fp.: 136-138° C.

Analog wurden dargestellt:

| Beispiel Nr. | Formel | Ausbeute (%) | Kp. (° C/mbar) oder Fp. (° C) |
|---|---|---|---|
| 34 | 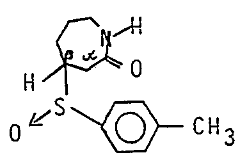 | 77 | 167-168 |
| 35 | 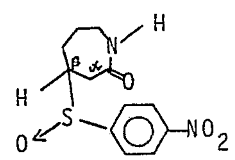 | 53 | 138-140 |
| 35a | 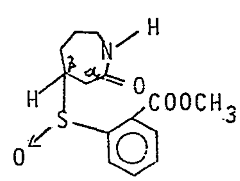 | 50 | Fp.: 133-136 |

*Beispiel 36:*

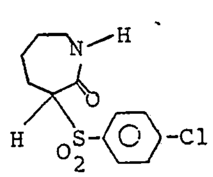

25,6 g (0,1 Mol) 3-[(4-Chlorphenyl)thio]azacycloheptan-2-on in 200 ml Chloroform werden bei Raumtemperatur mit 44 g m-Chlorperbenzoesäure (80-90%ig) portionsweise versetzt. Nach Abklingen der exothermen Reaktion wird noch 10 Std. bei Raumtemperatur gerührt und dann mit Natriumhydrogencarbonatlösung neutralisiert. Die organische Phase wird abgetrennt und eingeengt. Der kristalline Rückstand wird aus Methanol umkristallisiert.

Ausbeute: 24 g (= 83% d.Th.)
Fp.: 171° C

Analog wurden dargestellt:

| Beispiel Nr. | Formel | Ausbeute (%) | Kp. (° C/mbar) oder Fp. (° C) |
|---|---|---|---|
| 37 | 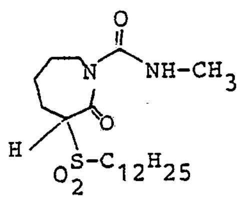 | 41 | 61 |
| 38 | 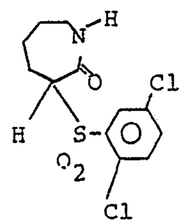 | 83 | 205-207 |
| 39 | 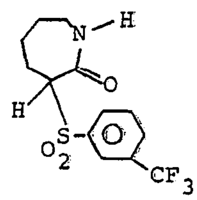 | 73 | 174-176 |
| 40 | 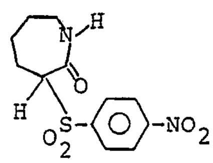 | 48 | 188-189 |
| 41 | 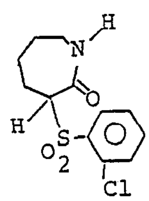 | 67 | 162-163 |
| 42 | 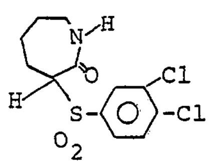 | 86 | 227-229 |

| Beispiel Nr. | Formel | Ausbeute (%) | Kp. (° C/mbar) oder Fp. (° C) |
|---|---|---|---|
| 43 | 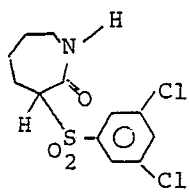 | 79 | 194-196 |
| 44 | 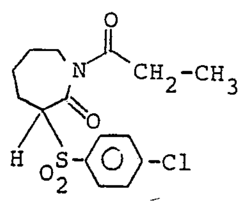 | 48 | 141-142 |
| 45 | 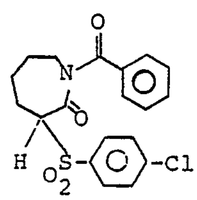 | 67 | 154-155 |
| 46 | 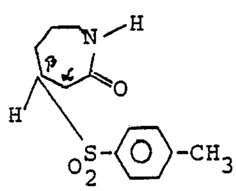 | 53 | 194-195 |
| 47 | 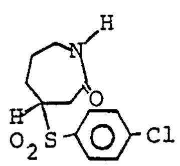 | 68 | 140-142 |
| 47a | 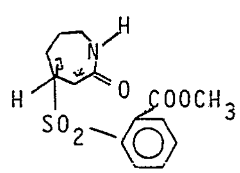 | 83 | 184-186 |

*Beispiel 48:*

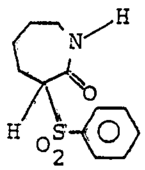

192 g α-Bromcaprolactam und 164 g Benzolsulfinsäure-Na-Salz werden in 600 ml Dimethylformamid 10 Std. Rückfluss gekocht. Anschliessend wird das ausgefallene Salz abgesaugt und die Mutterlauge im Vakuum eingeengt. Die ausgefallenen Kristalle werden aus Aceton umkristallisiert.
Ausbeute: 119 g (= 47% d.Th.)
Fp.: 180-181° C

Analog wurden dargestellt:

| Beispiel Nr. | Formel | Ausbeute (%) | Kp. (° C/mbar) oder Fp. (° C) |
|---|---|---|---|
| 49 | 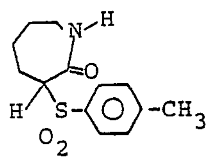 | 66 | 175 |
| 50 | 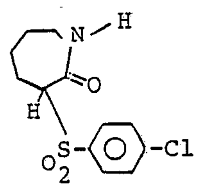 | 95 | 171 |

**Patentansprüche**

1. Sulfinyl- und Sulfonylazacycloheptan-2-one der allgemeinen Formel (I)

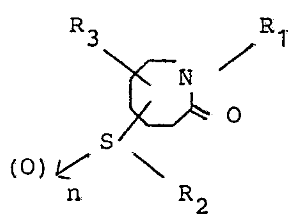

(I)

in welcher
n 1 oder 2 bedeutet,
$R_1$ für H, ggf. substituiertes $C_{1-4}$-Alkyl, ggf. substituiertes Phenyl, Acetyl, Propionly, Benzoyl und $C_{1-4}$-Alkylcarbamoyl steht,
$R_2$ ggf. substituiertes $C_{1-18}$-Alkyl, ggf. substituiertes $C_{2-6}$-Alkenyl, ggf. substituiertes $C_{3-10}$-Cycloalkyl, ggf. substituiertes Aralkyl mit 6 oder 10 C-Atomen im Arylteil und 1-4 C-Atomen im Alkylteil, ggf. substituiertes $C_{6-10}$-Aryl oder ggf. substituiertes Heterocyclyl mit 5-7 Ringatomen und 1-3 gleichen oder verschiedenen Heteroatomen bedeutet, wobei der Sulfinyl- bzw. Sulfonyrest sich in der α- bis δ-Position befinden kann,
$R_3$ für H oder $C_{1-4}$-Alkyl steht und die α- bis ε-Position einnehmen kann;
ggf. substituierte Alkyl-, Alkenyl-, Cycloalkyl-, Aralkyl-, Aryl- und Heterocyclylreste können einen oder mehrere, gleiche oder verschiedene Reste $R_4$ tragen, wobei $R_4$ für geradkettiges oder verzweigtes Alkyl mit 1 bis 6 C-Atomen und $CF_3$, $CCl_3$ sowie für Phenyl, $CH_3O$, $C_2H_5O$ sowie für Phenoxy, weiterhin für $CH_3S$, $C_2H_5S$, für $HCO-NH-$, für Dimethylamino, Diethylamino, für $CH_3OCO-$, $C_2H_5OCO-$, für Halogen, $-O\equiv N$, COOH und für $-NH_2$ sowie $NO_2$ steht.

2. Sulfinyl- und Sulfonylazacycloheptan-2-one nach Anspruch 1, gekennzeichnet durch die allgemeine Formel (Ia)

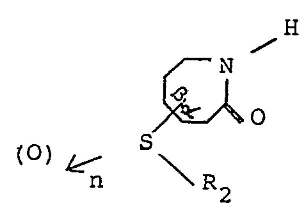

(Ia)

in welcher n und $R_2$ die in Anspruch 1 angegebene Bedeutung haben und sich der Sulfinyl- bzw. Sulfonylrest in der α- oder β-Position befindet.

3. 3-[(4-Chlorphenyl)sulfinyl]azacycloheptan-2-on.

4. 4-[(4-Chlorphenyl)sulfinyl]azacycloheptan-2-on.

5. 4-[(4-Methylphenyl)sulfonyl]azacycloheptan-2-on.

6. Verfahren zur Herstellung der Sulfinyl- und Sulfonylazacycloheptan-2-one der allgemeinen Formel (I)

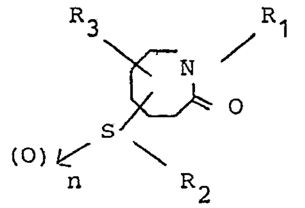

(I)

in welcher

n 1 oder 2 bedeutet,

$R_1$ für H, ggf. substituiertes $C_{1-4}$-Alkyl, ggf. substituiertes Phenyl, Acetyl, Propionyl, Benzoyl und $C_{1-4}$-Alkylcarbamoyl steht,

$R_2$ ggf. substituiertes $C_{1-18}$-Alkyl, ggf. substituiertes $C_{2-6}$-Alkenyl, ggf. substituiertes $C_{3-10}$-Cycloalkyl, ggf. substituiertes Aralkyl mit 6 oder 10 C-Atomen im Arylteil und 1-4 C-Atomen im Alkylteil, ggf. substituiertes $C_{6-10}$-Aryl oder ggf. substituiertes Heterocyclyl mit 5-7 Ringatomen und 1-3 gleichen oder verschiedenen Heteroatomen bedeutet, wobei der Sulfinyl- bzw. Sulfonylrest sich in der α- bis δ-Position befinden kann,

$R_3$ für H oder $C_{1-4}$-Alkyl steht und die α- bis ε-Position einnehmen kann;

ggf. substituierte Alkyl-, Alkenyl-, Cycloalkyl-, Aralkyl-, Aryl- und Heterocyclylreste können einen oder mehrere, gleiche oder verschiedene Reste $R_4$ tragen, wobei $R_4$ für geradkettiges oder verzweigtes Alkyl mit 1 bis 6 C-Atomen und $CF_3$, $CCl_3$ sowie für Phenyl, $CH_3O$, $C_2H_5O$ sowie für Phenoxy, weiterhin für $CH_3S$, $C_2H_5S$, für $HCO-NH-$, für Dimethylamino, Diethylamino, für $CH_3OCO-$, $C_2H_5OCO-$, für Halogen, $-C\equiv N$, COOH und für $-NH_2$ sowie $NO_2$ steht,

dadurch gekennzeichnet, dass man Thioether der Formel (II)

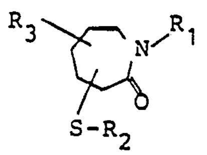

(II)

in welcher $R_1$, $R_2$ und $R_3$ die oben angegebene Bedeutung haben und der Sulfenylrest in α- bis δ-Position stehen kann, mit geeigneten Oxidationsmitteln, ggf. in Gegenwart von inerten Lösungsmitteln umsetzt.

7. Verfahren zur Herstellung von 3-Sulfonylazacycloheptan-2-onen der Formel (III)

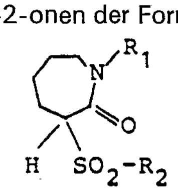

(III)

in welcher $R_1$ und $R_2$ die oben in Anspruch 1 angegebene Bedeutung besitzen, dadurch gekennzeichnet, dass man 3-Halogenazacycloheptan-2-one der Formel (IV)

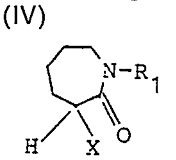

(IV)

in welcher $R_1$ die in Anspruch 1 angegebene Bedeutung besitzt und X für Halogen steht, mit Sulfinaten der Formel (V)

$$M^{\oplus}\ {}^{\ominus}O_2S-R_2 \qquad (V)$$

in welcher $R_2$ die in Anspruch 1 angegebene Bedeutung hat und $M^{\oplus}$ ein Alkalimetallkation darstellt, ggf. in Gegenwart von inerten Lösungsmitteln umsetzt.

8. Futterzusatzmittel enthaltend Sulfinyl- und Sulfonylazacycloheptan-2-one der Formel (I) in Anspruch 1.

9. Prämixe für die Tierfutterherstellung enthaltend Sulfinyl- und Sulfonylazacycloheptan-2-one der Formel (I) in Anspruch 1.

10. Verwendung von Sulfinyl- und Sulfonylazacycloheptan-2-onen der Formel (I) in Anspruch 1 in Futterzusatzmitteln und Prämixen für die Futterherstellung.

## Claims

1. Sulphinyl- and sulphonylazacycloheptan-2-ones of the general Formula (I):

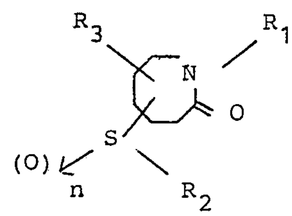

(I)

in which

n denotes 1 or 2,

$R_1$ represents H, optionally substituted $C_{1-4}$-alkyl, optionally substituted phenyl, acetyl, propionyl, benzoyl and $C_{1-4}$-alkylcarbamoyl,

$R_2$ denotes optionally substituted $C_{1-18}$-alkyl, optionally substituted $C_{2-6}$-alkenyl, optionally substituted $C_{3-10}$-cycloalkyl, optionally substituted aralkyl with 6 or 10 C atoms in the aryl part and 1-4 C atoms in the alkyl part, optionally substituted $C_{6-10}$-aryl or optionally substituted heterocyclyl with 5-7 ring atoms and 1-3 identical or different hetero atoms, it being possible for the sulphinyl or sulphonyl radical to be in the α to δ position,

$R_3$ represents H or $C_{1-4}$-alkyl and can assume the α to ε position,

optionally substituted alkyl, alkenyl, cycloalkyl, aralkyl, aryl and heterocyclyl radicals can carry one or more identical or different radicals $R_4$, $R_4$ representing straight-chain or branched alkyl with 1 to 6 C atoms and $CF_3$, $CCl_3$ and phenyl, $CH_3O$, $C_2H_5O$ and phenoxy, and also $CH_3S$, $C_2H_5S$, $HCO-NH-$, dimethylamino, diethylamino, $CH_3OCO-$, $C_2H_5OCO-$, halogen, $-CH\equiv N$, COOH and $-NH_2$ and $NO_2$.

2. Sulphinyl- and sulphonylazacycloheptan-2-ones according to Claim 1, characterised by the general Formula (Ia):

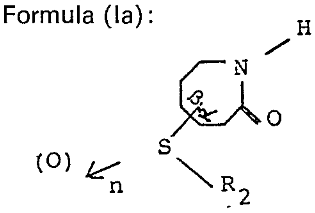

(Ia)

in which

n and $R_2$ have the meaning given in Claim 1 and the sulphinyl or sulphonyl radical is in the α or β position.

3. 3-[(4-Chlorphenyl)sulphinyl]azacycloheptan-2-one.

4. 4-[(4-Chlorphenyl)sulphinyl]azacycloheptan-2-one.

5. 4-[(4-Methylphenyl)sulphonyl]azacycloheptan-2-one.

6. Process for the preparation of the sulphinyl- and sulphonylazacycloheptan-2-ones of the general Formula (I):

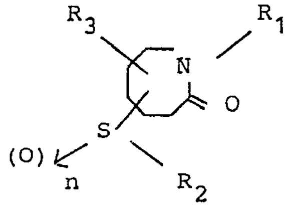
(I)

in which

n denotes 1 or 2,

$R_1$ represents H, optionally substituted $C_{1-4}$-alkyl, optionally substituted phenyl, acetyl, propionyl, benzoyl and $C_{1-4}$-alkylcarbamoyl,

$R_2$ denotes optionally substituted $C_{1-18}$-alkyl, optionally substituted $C_{2-6}$-alkenyl, optionally substituted $C_{3-10}$-cycloalkyl, optionally substituted aralkyl with 6 or 10 C atoms in the aryl part and 1-4 C atoms in the alkyl part, optionally substituted $C_{6-10}$-aryl or optionally substituted heterocyclyl with 5-7 ring atoms and 1-3 identical or different hetero atoms, it being possible for the sulphinyl or sulphonyl radical to be in the α to δ position,

$R_3$ represents H or $C_{1-4}$-alkyl and can assume the α to ε position,

optionally substituted alkyl, alkenyl, cycloalkyl, aralkyl, aryl and heterocyclyl radicals can carry one or more identical or different radicals $R_4$, $R_4$ representing straight-chain or branched alkyl with 1 to 6 C atoms and $CF_3$, $CCl_3$, and phenyl, $CH_3O$, $C_2H_5O$ and phenoxy, and also $CH_3S$, $C_2H_5S$, $HCO-NH-$, dimethylamino, diethylamino, $CH_3OCO$, $C_2H_5OCO$, halogen, $-C\equiv N$, COOH and $-NH_2$ and $NO_2$, characterised in that thioethers of the Formula (II):

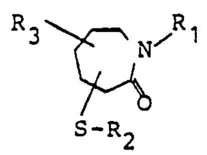
(II)

in which

$R_1$, $R_2$ and $R_3$ have the above-mentioned meaning and the sulphenyl radical can be in the α to δ position,

are reacted with suitable oxidising agents, if appropriate in the presence of inert solvents.

7. Process for the preparation of 3-sulphonylazacycloheptan-2-ones of the Formula (III):

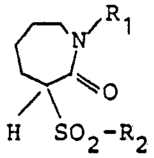
(III)

in which

$R_1$ and $R_2$ have the meaning given in Claim 1, characterised in that 3-halogenoazacycloheptan-2-ones of the Formula (IV):

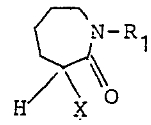
(IV)

in which

$R_1$ has the meaning given in Claim 1, and

X represents halogen,

are reacted with sulphinates of the Formula (V):

$$M^{\oplus}\ {}^{\ominus}O_2S-R_2 \qquad (V)$$

in which

$R_2$ has the meaning given in Claim 1, and

$M^{\oplus}$ represents an alkaline metal cation,

if appropriate in the presence of inert solvents.

8. Feed additives containing sulphinyl- and sulphonylazacycloheptan-2-ones of the Formula (I) in Claim 1.

9. Premixes for the production of animal feeds, containing sulphinyl- and sulphonylazacycloheptan-2-ones of the Formula (I) in Claim 1.

10. Use of sulphinyl- and sulphonylazacycloheptan-2-ones of the Formula (I) in Claim 1 in feed additives and premixes for the production of feeds.

**Revendications**

1. Sulfinyl- et sulfonylazacycloheptane-2-ones de formule générale (I):

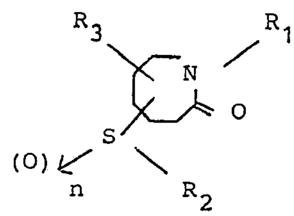
(I)

dans laquelle

n = 1 ou 2,

$R_1$ représente H, un groupe alkyle en $C_1$-$C_4$ éventuellement substitué, phényle éventuellement substitué, acétyle, propionyle, benzoyle et (alkyle en $C_1$-$C_4$)-carbamoyle,

$R_2$ représente un groupe alkyle en $C_1$-$C_{18}$ éventuellement substitué, alcényle en $C_2$-$C_6$ éventuellement substitué, cycloalkyle en $C_3$-$C_{10}$ éventuellement substitué, aralkyle contenant 6 ou 10 atomes de carbone dans la partie aryle et 1 à 4 atomes de carbone dans la partie alkyle, éventuellement substitué, aryle en $C_6$-$C_{10}$ éventuellement substitué ou un reste hétérocyclique contenant 5 à 7 atomes cycliques et 1 à 3 hétéroatomes identiques ou différents, éventuellement substitué, le reste sulfinyle ou sulfonyle pouvant se trouver dans la position α à δ,

$R_3$ représente H ou un groupe alkyle en $C_1$-$C_4$ et peut occuper la position α à ε.

Les restes alkyle, alcényle, cycloalkyle, aralkyle, aryle et les restes hétérocycliques éventuellement

substitués peuvent porter un ou plusieurs restes $R_4$ identiques ou différents, $R_4$ représentant un groupe alkyle droit ou ramifié en $C_1$-$C_6$ et $CF_3$, $CCl_3$ ou encore phényle, $CH_3O$, $C_2H_5O$ ou encore phénoxy, ou bien $CH_3S$, $C_2H_5S$, HCO−NH−, diméthylamino, diéthylamino, $CH_3OCO$−, $C_2H_5OCO$−, un halogène, un groupe −C≡N, COOH, $-NH_2$ ou encore $NO_2$.

2. Sulfinyl- et sulfonylazacycloheptane-2-ones selon la revendication 1, caractérisées en ce qu'elles répondent à la formule générale (Ia)

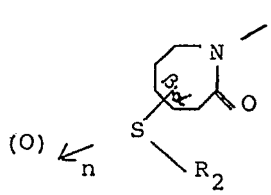

(Ia)

dans laquelle

n et $R_2$ ont les significations indiquées dans la revendication 1 et le reste sulfinyle ou sulfonyle se trouve en position α ou β.

3. La 3-[(4-chlorophényl)sulfinyl]azacycloheptane-2-one.

4. La 4-[(4-chlorophényl)sulfinyl]azacycloheptane-2-one.

5. La 4-[(4-méthylphényl)sulfonyl]azacycloheptane-2-one.

6. Procédé de préparation des sulfinyl- et sulfonylazacycloheptane-2-ones de formule générale (I):

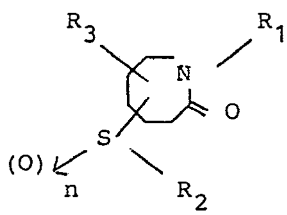

(I)

dans laquelle

n = 1 ou 2,

$R_1$ représente H, un groupe alkyle en $C_1$-$C_4$ éventuellement substitué, phényle éventuellement substitué, acétyle, propionyle, benzoyle et (alkyle en $C_1$-$C_4$)-carbamoyle,

$R_2$ représente un groupe alkyle en $C_1$-$C_{18}$ éventuellement substitué, alcényle en $C_2$-$C_6$ éventuellement substitué, cycloalkyle en $C_3$-$C_{10}$ éventuellement substitué, aralkyle contenant 6 ou 10 atomes de carbone dans la partie aryle et 1 à 4 atomes de carbone dans la partie alkyle, éventuellement substitué, aryle en $C_6$-$C_{10}$ éventuellement substitué ou un reste hétérocyclique contenant 5 à 7 atomes cycliques et 1 à 3 hétéroatomes identiques ou différents, éventuellement substitué, le reste sulfinyle ou sulfonyle pouvant se trouver dans la position α à δ,

$R_3$ représente H ou un groupe alkyle en $C_1$-$C_4$ et peut occuper la position α à ε;

les restes alkyle, alcényle, cycloalkyle, aralkyle, aryle et les restes hétérocycliques éventuellement substitués peuvent porter un ou plusieurs restes $R_4$ identiques ou différents, $R_4$ représentant un groupe alkyle droit ou ramifié en $C_1$-$C_6$ et $CH_3$, $CCl_3$ ou encore phényle, $CH_3O$, $C_2H_5O$ ou encore phénoxy, ou encore $CH_3S$, $C_2H_5S$, HCO−NH−, diméthylamino, diéthylamino, $CH_3OCO$−, $C_2H_5OCO$−, un halogène, un groupe −C≡N, COOH et $-NH_2$ ou $NO_2$, caractérisé en ce que l'on fait réagir des thioéthers de formule (II):

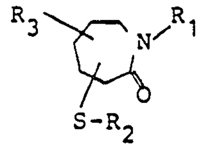

(II)

dans laquelle

$R_1$, $R_2$ et $R_3$ ont les significations indiquées ci-dessus et le reste sulfényle peut se trouver en position α à δ, avec des agents oxydants appropriés, éventuellement en présence de solvants inertes.

7. Procédé de préparation des 3-sulfonylazacycloheptane-2-ones de formule (III):

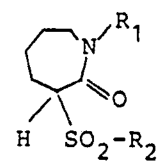

(III)

dans laquelle

$R_1$ et $R_2$ ont les significations indiquées dans la revendication 1, caractérisé en ce que l'on fait réagir des 3-halogénoazacycloheptane-2-ones de formule (IV):

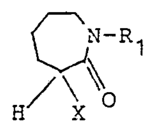

(IV)

dans laquelle

$R_1$ a les significations indiquées dans la revendication 1 et X représente un halogène, avec des sulfinates de formule (V):

$$M^{\oplus}\ {}^{\ominus}O_2S-R_2 \qquad (V)$$

dans laquelle

$R_2$ a les significations indiquées dans la revendication 1 et $M^{\oplus}$ représente un cation de métal alcalin, éventuellement en présence de solvants inertes.

8. Additif pour l'alimentation animale, contenant des sulfinyl- et sulfonylazacycloheptane-2-ones de formule (I) de la revendication 1.

9. Prémélanges pour la préparation d'aliments pour animaux, contenant des sulfinyl- et sulfonylazacycloheptane-2-ones de formule (I) de la revendication 1.

10. Utilisation des sulfinyl- et sulfonylazacycloheptane-2-ones de formule (I) de la revendication 1 dans des additifs à l'alimentation animale et des prémélanges pour la préparation d'aliments pour animaux.